# EUROPÄISCHE PATENTSCHRIFT

(11) **EP 1 711 472 B1**
(45) Veröffentlichungstag und Bekanntmachung des Hinweises auf die Patenterteilung: **25.05.2011**
(21) Anmeldenummer: 05701193.4
(22) Anmeldetag: 26.01.2005
(51) Int. Cl.: C07D 233/58

(54) **HERSTELLUNGSMETHODE FÜR IONISCHE FLÜSSIGKEITEN**
MEHTOD FOR PRODUCING IONIC LIQUIDS
PROCEDE DE FABRICATION DE LIQUIDES IONIQUES

(30) Priorität: 26.01.2004 DE 102004003958
(43) Veröffentlichungstag der Anmeldung: 18.10.2006
(73) Patentinhaber: BASF SE, 67056 Ludwigshafen (DE)
(72) Erfinder: MAASE, Matthias, 67346 Speyer (DE); MASSONNE, Klemens, 67098 Bad Dürkheim (DE); SZARVAS, Laszlo, 67071 Ludwigshafen (DE)
(86) Internationale Anmeldenummer: PCT/EP2005/000752
(87) Internationale Veröffentlichungsnummer: WO 2005/070896

(56) Entgegenhaltungen:
- EP-A- 0 291 074
- EP-A- 1 182 196
- EP-A- 1 182 197
- WO-A-01/40146
- WO-A-01/77081
- WO-A-2004/005222
- WO-A-2005/019183
- WASSERSCHEID P ET AL: "Ionische Flüssigkeiten, neue L sungen für die Übergangsmetallkatalyse" ANGEWANDTE CHEMIE, WILEY-VCH, WEINHEIM, DE, Nr. 112, 2000, Seiten 3926-3945, XP002271050 ISSN: 1433-7851 in der Anmeldung erwähnt
- WILKES J S ET AL: "AIR AND WATER STABLE 1-ETHYL-3-METHYLIMIDAZOLIUM BASED IONIC LIQUIDS" JOURNAL OF THE CHEMICAL SOCIETY, CHEMICAL COMMUNICATIONS, CHEMICAL SOCIETY. LETCHWORTH, GB, Nr. 13, 1992, Seiten 965-967, XP002044780 ISSN: 0022-4936 in der Anmeldung erwähnt

## Beschreibung

Die vorliegende Erfindung betrifft ein Verfahren zur Herstellung ionischer Flüssigkeiten.

Ionische Flüssigkeiten sind nach der Definition von Wasserscheid und Keim in: Angewandte Chemie 2000, 112, 3926-3945 bei relativ niedrigen Temperaturen schmelzende Salze mit nicht molekularem, ionischem Charakter. Sie sind bereits bei relativ niedrigen Temperaturen flüssig und dabei relativ niedrig viskos. Sie besitzen sehr gute Löslichkeiten für eine große Anzahl organischer, anorganischer und polymerer Substanzen. Darüber hinaus sind sie in der Regel nicht brennbar, nicht korrosiv und haben keinen messbaren Dampfdruck.

Ionische Flüssigkeiten sind Verbindungen, die aus positiven und negativen Ionen gebildet, jedoch insgesamt ladungsneutral sind. Die positiven wie auch die negativen Ionen sind überwiegend einwertig, möglich sind jedoch auch multivalente Anionen und/oder Kationen, beispielsweise mit einer bis fünf, vorzugsweise mit einer bis vier, besonders bevorzugt mit einer bis drei, und insbesondere mit einer bis zwei elektrischen Ladungen pro Ion. Die Ladungen können sich an verschiedenen lokalisierten oder delokalisierten Bereichen innerhalb eines Moleküls befinden, also betainartig, oder auch wie ein getrenntes Anion und Kation verteilt sein. Bevorzugt sind solche ionischen Flüssigkeiten, deren Verbindungen aus mindestens einem Kation und mindestens einem Anion aufgebaut sind.

Bekannte Einsatzgebiete für ionische Flüssigkeiten sind beispielsweise als Lösemittel für chemische Reaktionen, beispielsweise beschrieben in Peter Wasserscheid, Chemie in unserer Zeit, 37 (2003) Nr. 1, Seiten 52-63, als Hilfsmittel zur Abtrennung von Säuren aus chemischen Reaktionsgemischen, beispielsweise beschrieben in DE 102 02 838, als Hilfsstoffe für die Extraktivrektifikation zur Trennung engsiedender oder azeotroper Gemische, beispielsweise beschrieben in WO 02/074718, oder als Wärmeträger in solarthermischen Anlagen, beispielsweise beschrieben in Proceeding of Solar Forum, 21. bis 25. April 2001, Washington D.C. Die Verwendung von ionischen Flüssigkeiten als Extraktionsmittel zur Stofftrennung ist ferner in J. G. Huddleston et al., Chem. Commun. 1998, Seiten 1765-1766 erwähnt.

Beim Einsatz ionischer Flüssigkeiten ist deren Reinheit von großer Bedeutung. Verunreinigungen in ionischen Flüssigkeiten können beispielsweise den Verlauf chemischer Reaktionen negativ beeinflussen. So verweisen P. Teisen et al. in Electrochemical Society Proceedings, Vol. 99-41, Seiten 161-168 auf Probleme beim Einsatz von chloridhaltigen ionischen Flüssigkeiten in der Flüssigphasenhydrierung und bei der Übergangsmetall-katalysierten Suzuki-Reaktion, die auf Verunreinigungen zurückführen sind. Daher sind bei der Herstellung ionischer Flüssigkeiten hohe Anforderungen an die Reinheit der gewünschten Flüssigkeit zu stellen.

Die Synthese binärer ionischer Flüssigkeiten vom Typ [A]⁺[Y]⁻ kann beispielsweise durch ein zweistufiges Verfahren erfolgen (J. S. Wilkes, M. J. Zaworotko, J. Chem. Soc., Chem. Commun., 13, 1992, Seite 965). Dabei wird zunächst durch Reaktion eines Alkylierungsreagenzes LX und eines Amins NR¹R²R³ oder eines Phosphans PR¹R²R³ in einer Quarternisierungsreaktion das organische Ammoniumsalz [NR¹R²R³R]⁺X⁻ oder das organische Phosphoniumsalz [PR¹R²R³R]⁺X⁻ aufgebaut. X⁻ ist dabei im Allgemeinen ein Halogenidion. Das organische Halogenidsalz wird isoliert und in einer nachfolgenden, zweiten Reaktionsstufe in einer Austauschreaktion mit einem Alkali- oder Erdalkalisalz des Typs M⁺[Y]⁻ umgesetzt. Dies geschieht in einem Lösemittel, in dem das gebildete Nebenprodukt M⁺X⁻ schwer löslich, die zu synthetisierende ionische Flüssigkeit [A]⁺[Y]⁻ dagegen gut löslich ist.

Von Nachteil bei dieser Reaktionsführung ist, dass nur dann ein quantitativer Austausch des Halogenidsalzes [NR¹R²R³R]⁺X⁻ bzw. [PR¹R²R³R]⁺X⁻ zur gewünschten ionischen Flüssigkeit [NR¹R²R³R]⁺[Y]⁻ bzw. [PR¹R²R³R]⁺[Y]⁻ gelingt, wenn unter den Austauschbedingungen das Reaktionssystem vollständig wasserfrei ist. Diese Halogenidionen können - beispielsweise bei Verwendung der ionischen Flüssigkeit in Übergangsmetall-katalysierten Reaktionen - als Katalysatorgifte wirken. Darüber hinaus weist dieses Verfahren den Nachteil auf, dass das zunächst hergestellte Halogenidsalz stark hygroskopisch ist.

EP 1 182 197 beschreibt ein Verfahren zur Herstellung ionischer Flüssigkeiten der allgemeinen Formel [A]ₙ⁺[Y]ⁿ⁻, bei dem das zuvor beschriebene Verfahren angewendet und auf eine Isolierung der Zwischenprodukte verzichtet wird.

EP 1 182 196 beschreibt ein Verfahren zur Herstellung ionischer Flüssigkeiten der allgemeinen Formel [A]ₙ⁺[Y]ⁿ⁻ durch Alkylierung der zugrunde liegenden Amine, Phosphine, Imidazole, Pyrazole, Triazole oder Pyridine mit einem Disulfat der allgemeinen Formel R'-SO₄-R" und nachfolgendem Austausch des Sulfatanions R'-SO₄⁻ oder R"-SO₄⁻ durch das Anion [Y]⁻ oder [Y]²⁻.

Weitere Synthesewege für ionische Flüssigkeiten sind in "Ionic Liquids in Synthesis" von Peter Wasserscheid und Tom Welton, 2003, Wiley-VCH, 2003, Kapitel 2, Seiten 7 bis 17, beschrieben. Hierbei handelt es sich im Wesentlichen um die Fällung von Halogeniden ionischer Flüssigkeiten mit Silbersalzen, was jedoch aus ökonomischen Gründen von Nachteil ist. Auch die Herstellung von ionischen Flüssigkeiten durch Ionenaustausch an einem Harz ist teuer und nur aufwendig realisierbar. Hinzukommend erfordert die Verwendung von Ionenaustaucherharzen deren Regeneration. Der ebenfalls bekannte Austausch von Halogeniden ionischer Flüssigkeiten zu deren Modifizierung durch Waschen mit Wasser ist schließlich auf hydrophobe, nicht mit Wasser mischbare ionische Flüssigkeiten, die eher die Ausnahme sind, beschränkt.

WO 2005/019183 offenbart ein Verfahren zur Herstellung von 1,3-substituierten Imidazolium-Salzen durch Umsetzung von 1,3-substituierten Imidazolium-Salzen mit einer starken Base bei einer Temperatur im Bereich von 20 bis 250 °C unter Abdestillation des gebildeten 1,3-substituierten Imidazol-2-ylidens. Dieses wird anschließend im gasförmigen Zustand mit einer Protonensäure in Kontakt gebracht.

WO 01/77081 offenbart ein Verfahren zur Herstellung von Imidazolium-Carbenen umfassend das Erhitzen eines Imidazolium-Halogenids mit einer starken Base unter reduziertem Druck und Isolierung des entstehenden Imidazol-2-ylidens durch Destillation. Dieses wird dann mit einer starken Säure in das entsprechende Salz überführt.

EP 0 291 074 A offenbart ein Verfahren zur Herstellung von hochreinen quartemären Salzen durch Umsetzung eines tertiären Amins oder Phosphins mit einem Carbonsäurediester, um das entsprechende quartemäre Carbonat zu erhalten und weitere Umsetzung mit einer Säure, um eine Decarboxylierung hervorzurufen.

Aufgabe der vorliegenden Erfindung ist es dementsprechend ein allgemein anwendbares Verfahren zur Modifizierung bzw. Herstellung ionischer Flüssigkeiten zur Verfügung zu stellen, welches die zuvor diskutierten Nachteile nicht aufweist und zu Produkten führt, welche in hoher Reinheit und Ausbeute als unmittelbares Verfahrensprodukt erhalten werden.

Die Lösung der vorliegenden Aufgabe geht aus von einem Verfahren zur Modifizierung ionischer Flüssigkeiten, die als Kation ein Phosphonium- und/oder Ammoniumkation, das aus der nachfolgend dargestellten Gruppe ausgewählt ist, nämlich aus
- quartemären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 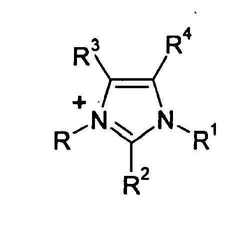 sowie allen dieser Formel analogen isomeren Imidazoliniumkationen und Imidazolidiniumkationen,
- H-Pyrazolium-Kationen der allgemeinen Formel 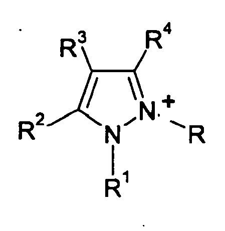 sowie 3H-Pyrazolium-Kationen, 4H-Pyrazolium-Kationen, 1-Pyrazolinium-Kationen, 2-Pyrazolinium-Kationen und 3-Pyrazolinium-Kationen,
- Pyridinium-Kationen der allgemeinen Formel 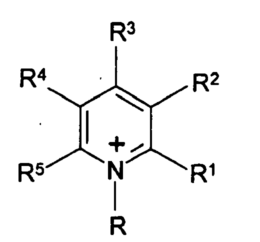 sowie Pyridazinium-, Pyrimidinium- und Pyrazinium-Kationen,
- Pyrrolidinium-Kationen der allgemeinen Formel 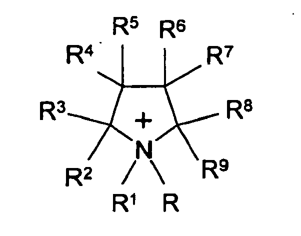
- fünf- bis mindestens sechsgliedrigen heterocyclischen Kationen, die mindestens ein Phosphor- oder Stickstoffatom sowie gegebenenfalls ein Sauerstoff- oder Schwefelatom aufweisen, beispielsweise Thiazolium-, Oxazolium-, 1,2,4-Triazolium- oder 1,2,3-Triazolium-Kationen, vorzugsweise solchen Verbindungen, die mindestens einen fünf- bis sechsgliedrigen Heterocyclus enthalten, der ein, zwei oder drei Stickstoffatome und ein Schwefel- oder ein Sauerstoffatom aufweist, besonders bevorzugt solchen Verbindungen mit ein oder zwei Stickstoffatomen,
- Triazol-Kationen der allgemeinen Formel 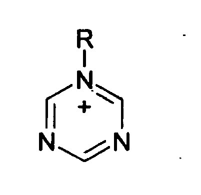 wobei der Triazol-Kern substituiert sein kann mit wenigstens einer Gruppe, die ausgewählt ist aus der Gruppe bestehend aus C₁-C₆-Alkyl-, C₁-C₆-Alkoxy-, Amino-C₁-C₆-alkyl-, C₅-C₁₂-Aryl- oder C₅-C₁₂-Aryl-C₁-C₆-alkylgruppen,
- dem 1,8-Diazabicyclo[5.4.0]undec-7-enium-Kation sowie dem 1,8-Diazabicyclo-[4.3.0]non-5-enium-Kation 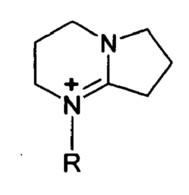
- Chinolinium-Kationen der allgemeinen Formel 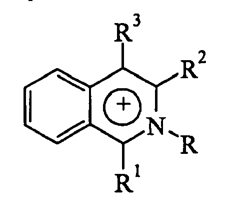
- Thiazolium-Kationen der allgemeinen Formel 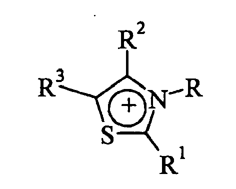
- Triazinium-Kationen der allgemeinen Formel 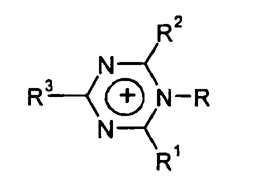 sowie Oligo- und Polymere, die diese Kationen enthalten, wobei die Reste R, R¹, R², R³, R⁴, R⁵, R⁶, R⁷, R⁸ und R⁹ unabhängig voneinander jeweils Wasserstoff, C₁-C₁₈-Alkyl, C₆-C₁₂=Aryl, C₅-C₁₂-Cycloalkyl, gegebenenfalls durch ein oder mehrere Sauerstoff- und/oder Schwefelatome und/oder ein oder mehrere gegebenenfalls substituierte Iminogruppen unterbrochenes C₂-C₁₈-Alkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können und ein Anion, das ausgewählt ist aus der Gruppe, bestehend aus Halogeniden, Arylsulfonaten, Alkylsulfonaten, Sulfat, Hydrogensulfat, Alkylsulfaten, Hydrogencarbonat, Carbonat, Triflaten und Carboxylaten, enthält. Das erfindungsgemäße Verfahren ist dann dadurch gekennzeichnet, dass diese ionischen Flüssigkeiten in einem ersten Verfahrensschritt mit einem Alkoholat oder Bariumhydroxid bei 10 bis 90 °C umgesetzt werden, wobei stark basische ionische Flüssigkeiten resultieren, und die stark basischen ionischen Flüssigkeiten in einem zweiten Verfahrensschritt mit einer Säure neutralisiert werden.

Im Rahmen der vorliegenden Erfindung wird unter dem Begriff "Modifizierung ionischer Flüssigkeiten" im Allgemeinen ein Austausch (Substitution) des Anions der ionischen Flüssigkeit verstanden.

Die in dem erfindungsgemäßen Verfahren verwendete, zu modifizierende ionische Flüssigkeit ist vorzugsweise präparativ leicht zugänglich. Geeignete ionische Flüssigkeiten weisen demnach vorzugsweise die allgemeine Formel (I)

[Q⁺]ₙ [Z]ⁿ⁻ (I)

auf, wobei
n =1, 2, 3 oder 4 ist und
das Kation [Q⁺] ein Phosphonium- und/oder Ammonium-Kation ist, das aus der nachfolgend dargestellten Gruppe ausgewählt ist, nämlich aus
- quartemären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 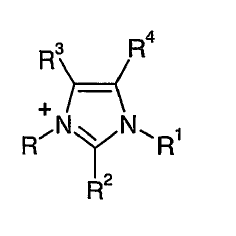 sowie allen dieser Formel analogen isomeren Imidazoliniumkationen und Imidazolidiniumkationen,
- H-Pyrazolium-Kationen der allgemeinen Formel 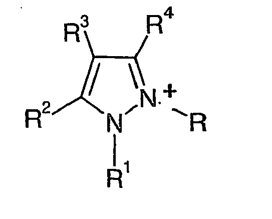 sowie 3H-Pyrazolium-Kationen, 4H-Pyrazolium-Kationen, 1-Pyrazolinium-Kationen, 2-Pyrazolinium-Kationen und 3-Pyrazolinium-Kationen,
- Pyridinium-Kationen der allgemeinen Formel 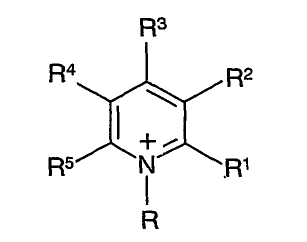 sowie Pyridazinium-, Pyrimidinium- und Pyrazinium-Kationen,
- Pyrrolidinium-Kationen der allgemeinen Formel 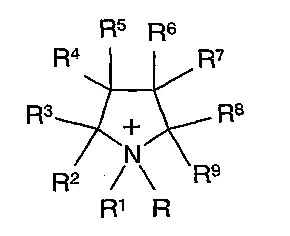
- fünf- bis mindestens sechsgliedrigen heterocyclischen Kationen, die mindestens ein Phosphor- oder Stickstoffatom sowie gegebenenfalls ein Sauerstoff- oder Schwefelatom aufweisen, beispielsweise Thiazolium-, Oxazolium-, 1,2,4-Triazolium- oder 1,2,3-Triazolium-Kationen, vorzugsweise solchen Verbindungen, die mindestens einen fünf- bis sechsgliedrigen Heterocyclus enthalten, der ein, zwei oder drei Stickstoffatome und ein Schwefel- oder ein Sauerstoffatom aufweist, besonders bevorzugt solchen Verbindungen mit ein oder zwei Stickstoffatomen,
- Triazol-Kationen der allgemeinen Formel 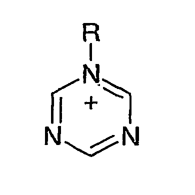 wobei der Triazol-Kern substituiert sein kann mit wenigstens einer Gruppe, die ausgewählt ist aus der Gruppe bestehend aus C₁-C₆-Alkyl-, C₁-C₆-Alkoxy-, Amino-C₁-C₆-alkyl-, C₅-C₁₂-Aryl- oder C₅-C₁₂-Aryl-C₁-C₆-alkylgruppen,
- dem 1,8-Diazabicyclo[5.4.0]undec-7-enium-Kation sowie dem 1,8-Diazabicyclo-[4.3.0]non-5-enium-Kation 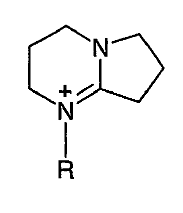
- Chinolinium-Kationen der allgemeinen Formel 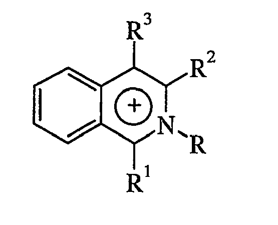
- Thiazolium-Kationen der allgemeinen Formel 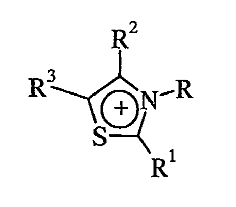
- Triazinium-Kationen der allgemeinen Formel 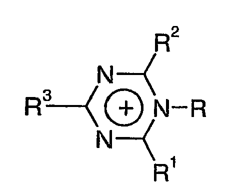 sowie Oligo- und Polymere, die diese Kationen enthalten, wobei die Reste R, R¹, R², R³, R⁴, R⁵, R⁶, R⁷, R⁸ und R⁹ unabhängig voneinander jeweils Wasserstoff, C₁-C₁₈-Alkyl, C₆-C₁₂-Aryl, C₅-C₁₂-Cycloalkyl, gegebenenfalls durch ein oder mehrere Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

Die in dem erfindungsgemäßen Verfahren als Edukt verwendete, zu modifizierende ionische Flüssigkeit enthält als Kation vorzugsweise ein heterocyclisches Kation, besonders bevorzugt ein Imidazolium-, ein Pyridinium- oder ein Phosphonium-Kation, insbesondere ein Imidazolium-Kation, speziell ein 1,3-substituiertes Imidazoliumkation, beispielsweise 1,3-Dimethylimdazolium, 1-Ethyl-3-methylimi-dazolium, 1-Methyl-3-propylimidazolium, 1-Isopropyl-3-methylimidazolium, 1-Butyl-3-methylimidazolium, 1-Methyl-3-pentylimidazolium, 1-Hexyl-3-methylimidazolium, 1-Heptyl-3-methylimidazolium, 1-Methyl-3-octylimidazolium, 1-Decyl-3-methylimidazo-lium, 1-Methyl-3-benzylimidazolium, 1-Methyl-3-(3-phenylpropyl)imidazolium, 1-(2-Ethyl)hexyl-3-methylimidazolium, 1-Methyl-3-nonylimidazolium, 1-Methyl-3-decylimida-zolium, 1,2,3 Trimethylimidazolium, 1-Ethyl-2,3-dimethylimidazolium oder 1-Butyl-2,3-dimethylimidazolium enthalten.

Die im erfindungsgemäßen Verfahren verwendeten ionischen Flüssigkeiten enthalten ein Anion [Z]ⁿ⁻, wobei dieses für das teil- oder volldeprotonierte Anion einer anorganischen oder organischen Protonensäure HₙA steht, wobei n eine ganze, positive Zahl ist und den Ladungszustand des Anions wiedergibt.

Das Anion [Z]ⁿ⁻ der in dem erfindungsgemäßen Verfahren verwendeten, zu modifizierende ionischen Flüssigkeit ist ausgewählt aus der Gruppe bestehend aus Halogeniden, Arylsulfonaten, Alkylsulfonaten, Sulfat, Hydrogensulfat, Alkylsulfaten, Hydrogencarbonat, Carbonat, Alkylcarbonate, Triflaten und Carboxylaten. Dabei sind Anionen [Z]ⁿ⁻ bevorzugt, die mit den verwendeten Kationen der Alkoholate oder Hydroxide schwer lösliche Salze bilden.

In dem ersten Verfahrensschritt des erfindungsgemäßen Verfahrens wird die zuvor beschriebene, zu modifizierende ionische Flüssigkeit mit einem Alkoholat oder Bariumhydroxid umgesetzt. Hierbei bildet sich eine stark basische ionische Flüssigkeit der allgemeinen Formel (II)

[Q⁺]ₙ[X]ⁿ⁻ (II)

mit
[X]ⁿ⁻ =OH⁻ und Alkoholate.

Der pKₛ-Wert der korrespondierenden Säure des Anions der stark basischen ionischen Flüssigkeit ist vorzugsweise größer 1,9, besonders bevorzugt größer 3, insbesondere größer 4, speziell größer 7.

Die Kationen der verwendeten Alkoholate sind vorzugsweise so ausgewählt, dass das in dem ersten Verfahrensschritt gebildete Salz aus dem Kation und dem Anion [Z]ⁿ⁻ in dem verwendeten Lösemittel schwer löslich ist und ausfällt. Geeignete Kationen sind beispielsweise Alkali-, Erdalkalikationen und Ammoniumkationen, besonders bevorzugt Li⁺, Na⁺, K⁺, Rb⁺, NH₄⁺, Ca²⁺, Ba²⁺ und Mg²⁺, insbesondere Li⁺, Na⁺, K⁺ und Ba²⁺, speziell K⁺, Na⁺ und Ba²⁺.

Falls in dem ersten Verfahrensschritt des erfindungsgemäßen Verfahrens ein Alkoholat verwendet wird, so ist dieses Alkoholat vorzugsweise ausgewählt aus der Gruppe bestehend aus Alkoholaten RO⁻ mit R = C₁-C₁₈-Alkyl, C₆-C₁₂-Aryl und C₅-C₁₂-Cycloalkyl. Besonders bevorzugt wird das Alkoholat ausgewählt aus der Gruppe, bestehend aus tert.-Butanol, n-Butanolat, iso-Propanolat, n-Propanolat, iso-Butanolat, Ethanolat, Methanolat, n-Pentanolat, iso-Pentanolat, 2-Ethylhexanolat, 2-Propylheptanolat, Nonanolat, Octanolat, Decanolat und Isomeren der zuvor erwähnten Alkoholate. Insbesondere wird das Alkoholat ausgewählt aus der Gruppe, bestehend aus tert.-Butanolat, iso-Propanolat, Ethanolat und Methanolat. Speziell ist das Alkoholat tert.-Butanolat oder Methanolat.

Falls in dem erfindungsgemäßen Verfahren die verwendete, zu modifizierende ionische Flüssigkeit mit einem Alkoholat umgesetzt wird, so findet diese Umsetzung vorzugsweise in einem alkoholischen Lösemittel statt. Geeignet sind beispielsweise tert.-Butanol, iso-Butanol, iso-Propanol, Ethanol, Methanol, n-Butanol, n-Propanol, n-Pentanol, iso-Pentanol, 2-Ethylhexanol, 2-Propylheptanol, Nonanol, Octanol, Isomerengemische und Gemische der zuvor erwähnten Alkohole. Besonders bevorzugt sind tert.-Butanol, iso-Propanol, Ethanol und Methanol. Insbesondere bevorzugt sind tert.-Butanol und Methanol.

Die Umsetzung kann aber auch in konventionellen Lösemitteln durchgeführt werden, beispielsweise Ether, wie Methyl-tert.-butylether, Tetrahydrofuran, Dioxan oder Glyme; Aceton, Acetonitril, Dimethylformamid, Dimethylsulfoxid, Chloroform, Methylenchlorid, Diethylacetamid, Diethylketon, Methylethylketon, Dimethylharnstoff oder Amine, wie Triethylamin, Pyridin oder Pyrrolidin.

Die Umsetzung kann auch in Mischungen der zuvor erwähnten Alkohole und konventionellen Lösemitteln - sofern diese miteinander mischbar sind - durchgeführt werden. Gegebenenfalls kann die Umsetzung in dem ersten Verfahrensschritt auch direkt in dem Alkoholat oder Hydroxid durchgeführt werden, sofern dieses flüssig ist.

In einer bevorzugten Ausführungsform der vorliegenden Erfindung findet die Umsetzung in Gegenwart einer Säure als Lösemittel statt, die in dem zweiten Verfahrensschritt zur Einführung des gewünschten Anions in die ionische Flüssigkeit verwendet wird. In dieser Ausführungsform entfällt die Zugabe der Säure im zweiten Verfahrensschritt vorzugsweise.

Wenn in dem erfindungsgemäßen Verfahren die verwendete, zu modifizierende ionische Flüssigkeit mit Bariumhydroxid umgesetzt wird, so findet diese Umsetzung vorzugsweise in Wasser als Lösemittel statt. Allerdings können auch die zuvor diskutierten konventionellen Lösemittel, gegebenenfalls in Mischung mit Wasser, verwendet werden.

Die Verwendung von Bariumhydroxid ist besonders bevorzugt, wenn als Edukt ionische Flüssigkeiten verwendet werden, die ein Sulfatanion oder Hydrogensülfatanion aufweisen, da sich das schwer lösliche BaSO₄ bildet. Als Bariumhydroxid wird vorzugsweise Ba(OH)₂. 8 H₂O verwendet.
Die Umsetzung der verwendeten, zu modifizierenden ionischen Flüssigkeit mit dem Alkoholat oder dem Bariumhydroxid erfolgt bei Temperaturen von 10 bis 90 °C, insbesondere 30 bis 85 °C. Die Umsetzungsdauer beträgt dabei vorzugsweise 1 bis 16 h, besonders bevorzugt 30 min bis 3 h, insbesondere 10 min bis 2 h. Da Bariumhydroxid in der Mehrzahl der verwendeten Lösemittel nur mäßig löslich ist, kann es bevorzugt sein, dass die erfindungsgemäße Umsetzung unter Einsatz von Bariumhydroxid in Gegenwart der korrespondierenden Säure des Anions [A]ⁿ⁻, das in die ionische Flüssigkeit eingeführt werden soll, durchgeführt wird. Besonders bevorzgut ist dabei, dass 1 Äquivalent der korrespondierenden Säure verwendet wird, so dass sich zunächst ein besser lösliches gemischtes Bariumsalz bildet, das mit der zu modifizierenden ionischen Flüssigkeit umgesetzt wird.

Beim Einsaz von Bariumhydroxid oder von gemischten Bariumsalzen kann die ionische Flüssigkeit durch deren Zugabe in eine Lösung der Bariumhydroxids oder des gemischten Bariumsalzes umgesetzt werden. Alternativ ist es auch möglich, dass das Bariumhydroxid oder das gemischte Bariumsalz vorgelegt wird und die verwendete, zu modifizeirende ionische Flüssigkeit, gegebenenfalls in Wasser und/oder einem der zuvor beschriebenen konventionellen Lösemittel gelöst, zugegeben wird.

In dem ersten Verfahrensschritt bildet sich durch die Umsetzung der verwendeten, zu modifizierenden ionischen Flüssigkeit mit dem Alkoholat oder mit dem Bariumhydroxid gegebenenfalls ein schwerlöslicher Niederschlag, beispielsweise von Alkali- oder Erdalkalihalogeniden bei Verwendung von entsprechenden Alkoholaten und ionischen Flüssigkeiten, die Halogenidionen enthalten, oder von Bariumsulfat bei Verwendung von Bariumhydroxid und ionischen Flüssigkeiten, die Sulfationen oder Hydrogensulfatanionen enthalten. Daher wird nach dem ersten Verfahrensschritt der ausgefallene Feststoff gegebenenfalls abgetrennt.

Die in dem ersten Verfahrensschritt erhaltene ionische Flüssigkeit [Q⁺]ₙ[X⁻]ⁿ⁻ kann vor dem zweiten Verfahrensschritt gegebenenfalls isoliert werden.

In dem zweiten Verfahrensschritt des erfindungsgemäßen Verfahrens wird die erhaltene stark basische ionische Flüssigkeit mit einer Säure [H⁺]ₙ[A]ⁿ⁻ neutralisiert. Hierfür geeignete Säuren weisen einen kleineren pKₛ-Wert als die korrespondierenden Säuren des Anions [X]ⁿ⁻ auf.

Als Säuren kommen hierfür Brönsted- und Lewis-Säuren in Betracht. Welche Säuren als Brönsted- oder Lewissäuren bezeichnet werden, wird in Hollemann-Wiberg, Lehrbuch, der Anorganischen Chemie, 91.-100. Auflage, Walter de Gruyter, Berlin New York 1985, S. 235 bzw. S. 239 beschrieben. Zu den Lewissäuren im Sinnen dieser Erfindung zählen auch die als Friedel-Crafts-Katalysatoren verwendeten Lewissäuren, die in George A. Olah, Friedel-Crafts and Releated Reactions, Vol. I, 191 bis 197, 201 und 284-90 (1963) beschrieben sind. Als Beispiele genannt seien Aluminiumtrichlorid (AlCl₃), Eisen(III)chlorid (FeCl₃), Aluminiumtribromid (AlBr₃) und Zinchlorid (ZnCl₂).

Allgemein enthalten die erfindungsgemäß zu verwendenden Lewis-Säuren kationische Formen der Metalle der Gruppen Ib, IIb, IIIa, IIIb, IVa, IVb, Va, Vb, VIb, VIIb und VIII des Periodensystems der Elemente sowie der seltenen Erden, wie beispielsweise Lanthan, Cer, Praseodym, Neodym, Samarium, Europium, Gadolinium, Terbium, Dysprosium, Holmium, Erbium, Thulium, Ytterbium oder Lutetium.

Genannt seien besonders Zink, Cadmium, Beryllium, Bor, Aluminium, Gallium, Indium, Thallium, Titan, Zirkonium, Hafnium, Erbium, Germanium, Zinn, Vanadium, Niob, Skandium, Yttrium, Chrom, Molybdän, Wolfram, Mangan, Rhenium, Palladium, Thorium, Eisen, Kupfer und Kobalt. Bevorzugt sind Bor, Zink, Cadmium, Titan, Zinn, Eisen, Kobalt.

Als Gegenionen der Lewis-Säure kommen in Frage F, Cl⁻, ClO₃⁻, ClO₄⁻, Br⁻, r, IO₃-, CN⁻, OCN⁻, SCN⁻, NO₂⁻, NO₃⁻, HCO₃⁻, HCO₃⁻, CO₃²⁻, S²⁻, SH⁻, HSO₃⁻, SO₃²⁻, HSO⁴⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, Dithiocarbamat, Salicylat, (OCₙH2ₙ₊₁)⁻, (CₙH₂ₙ₋₁O₂)⁻, (CₙH₂ₙ₋₃O₂⁻ sowie (Cₙ₊₁H₂ₙ₋₂O₄)²⁻, wobei n für 1 bis 20 steht, Methansulfonat (CH₃SO₃⁻), Trifluormethansulfonat (CF₃SO₃⁻), Toluolsulfonat (CH₃C₆H₄SO₃⁻), Benzolsulfonat (C₆H₅SO₃⁻), Hydroxid (OH⁻), Anionen aromatischer Säuren wie Benzoesäure, Phtalsäure, und dergleichen und 1,3-Dicarbonylverbindungen.

Weiterhin genannt seien Carboxylate, insbesondere sind zu erwähnen Formiat, Acetat, Trifluoracetat, Propionat, Hexanoat und 2-Ethylhexanoat, Stearat sowie Oxalat, Acetylacetonat, Tartrat, Acrylat und Methacrylat, bevorzugt Formiat, Acetat, Propionat, Oxalat, Acetylacetonat, Acrylat und Methacrylat.

Weiterhin kommen als Lewis-Säuren borhaltige Verbindungen der allgemeinen Formel BR'ₙ (OR")ₘ mit n = 0, 1, 2, 3 und m = 3-n in Betracht, worin R' und R", jeweils unabhängig voneinander, Wasserstoff, C₁-C₁₈-Alkyl, C₆-C₁₂-Aryl, C₅-C₁₂-Cycloalkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein kann. Die Reste R' können auch miteinander verbunden sein.

Als bevorzugte Beispiele für Lewis-Säuren seien neben den oben angeführten AlCl₃, FeCl₃, AlBr₃ und ZnCl₂ genannt BeCl₂, ZnBr₂, Znl₂, ZnSO₄, CuCl₂, CuCl, Cu(O₃SCF₃)₂, CoCl₂, Col₂, Fel₂, FeCl₂, FeCl₂(THF)₂, TiCl₄(THF)₂, TiCl₄, TiCl₃, ClTi(OiPr)₃, SnCl₂, SnCl₄, Sn(SO₄), Sn(SO₄)₂, MnCl₂, MnBr₂, ScCl₃, BPh₃, BCl₃, BBr₃, BF₃ · OEt₂, BF₃ · OMe₂, BF₃ · MeOH, BF₃ · CH₃COOH, BF₃ · CH₃CN, B(CF₃COO)₃, B(OEt)₃, B(OMe)₃, B(O/Pr)₃, PhB(OH)₂, PhB(OR)₂ (mit R = H, Alkyl), 3-MeO-PhB(OH)₂, 4-MeO-PhB(OH)₂, 3-F-PhB(OH)₂, 4-F-PhB(OH)₂, (C₂H₅)₃Al, (C₂H₅)₂AlCl, (C₂H₅)AlCl₂, (C₈H₁₇)AlCl₂, (C8H17)2AlCl, (iso-C₄H₉)₂AlCl, Ph₂AlCl, PhAlCl₂, Al(acac)₃, Al(O/Pr)₃, Al(O*n*Bu)3, Al(O*sek*Bu)₃, Al(OE)₃, GaCl₃, ReCl₅, ZrCl₄, NbCl₅, VCl₃, CrCl₂, MoCl₅, YCl₃, CdCl₂, CdBr₂, SbCl₃, SbCl₅, BiCl₃, ZrCl₄, UCl₄, LaCl₃, CeCl₃, Er(O₃SCF₃), Yb(O₂CCF₃)₃, SmCl₃, Sml₂, B(C₆H₅)₃ und TaCl₅.

Die Lewis-Säuren können durch Alkali- oder Erdalkalimetallhalogenide, beispielsweise LiCl oder NaCl, stabilisiert sein. Dazu werden die (Erd-)Alkalimetallhalogenide zur Lewis-Säure im molaren Verhältnis 0-100:1 gemischt.

Weitere geeignete Säuren sind beispielsweise Iodwasserstoff (HI), Fluorwasserstoff (HF), Chlorwasserstoff (HCI), Salpetersäure (HNO₃), salpetrige Säure (HNO₂), Bromwasserstoffsäure (HBr), Kohlensäure (H₂CO₃), Methylkohlensäure (HO(CO)OCH₃), Ethylkohlensäure (HO(CO)OC₂H₅), n-Butylkohlensäure, Schwefelsäure (H₂SO₄⁻), Hydrogensulfat (HSO₄⁻), Methylschwefelsäure (HO(SO₂)OCH₃), Ethylschwefelsäure (HO(SO₂)OC₂H₅), Phosphorsäure (H₃PO₄), Dihydrogenphosphat (H₂PO₄⁻), Ameisensäure (HCOOH), Essigsäure (CH₃COOH), Propionsäure, n- und iso-Buttersäure, Pivalinsäure, para-Toluolsulfonsäure, Benzolsulfonsäure, Benzoesäure, 2,4,6-Trimethylbenzoesäure, Mandelsäure, Methansulfonsäure oder Trifluormethansulfonsäure.

Weitere geeignete Säuren können von den nachfolgenden Anionen [A]ⁿ⁻ abgeleitet werden, wobei das Anion ausgewählt ist aus der Gruppe bestehend aus
- den Halogeniden, halogenhaltigen Verbindungen bzw. Pseudohalogeniden der Formel:
   Br⁻, BF₄⁻, PF₆⁻, AlCl₄⁻, Al₂Cl₇⁻, FeCl₄⁻, BCl₄⁻, SbF₆⁻, AsF₆⁻, ZnCl₃⁻, SnCl₃⁻, CF₃SO₃⁻, (CF₃SO₃)₂N⁻, CF₃CO₂⁻, CCl₃CO₂⁻, CN, SCN, OCN
- den Sulfaten, Sulfiten oder Sulfonaten der allgemeinen Formel:
   SO₄²⁻, HSO₄⁻, SO₃²⁻, HSO₃⁻, R^{a}OSO₃⁻, R^{a}SO₃⁻.
- den Phosphaten der allgemeinen Formel
   PO₄³⁻, HPO₄²⁻, H₂PO₄⁻, R^{a}PO₄²⁻, HR^{a}PO₄⁻, R^{a}R^{b}PO₄⁻
- den Phosphonaten oder den Phosphinaten der allgemeinen Formel
   R^{a}HPO₃⁻,R^{a}R^{b}PO₂⁻, R^{a}R^{b}PO₃⁻
- den Phosphiten der allgemeinen Formel
   PO₃³⁻, HPO₃²⁻, H₂PO₃⁻, R^{a}PO₃²⁻, R^{a}HPO₃⁻, R^{a}R^{b}PO₃⁻
- den Phosphoniten oder den Phosphiniten der allgemeinen Formel
   R^{a}R^{b}PO₂⁻, R^{a}HPO₂⁻, R^{a}R^{b}PO⁻, R^{a}HPO⁻
- den Carbonsäuren der allgemeinen Formel
   R^{a}COO⁻
- den Boraten der allgemeinen Formel
   BO₃³⁻, HBO₃²⁻, H₂BO₃⁻, R^{a}R^{b}BO₃⁻, R^{a}HBO₃⁻, R^{a}BO₃²⁻, [BR^{a}R^{b}R^{c}R^{d}]⁻
- den Boronaten der allgemeinen Formel
   R^{a}BO₂²⁻, R^{a}R^{b}BO⁻
- den Carbonaten oder den Kohlensäureestern der allgemeinen Formel
   HCO₃⁻, CO₃²⁻, R^{a}CO₃⁻
- den Silikaten oder den Kieselsäuresäureestern der allgemeinen Formel
   SiO₄⁴⁻, HSiO₄³⁻, H₂SiO₄²⁻, H₃SiO₄⁻, R^{a}SᵢO₄³⁻, R^{a}R^{b}SiO₄²⁻, R^{a}R^{b}R^{c}SiO₄⁻, HR^{a-} SiO₄²⁻, H₂R^{a}SiO₄⁻, HR^{a}R^{b}SiO₄⁻
- den Alkyl- bzw. Arylsilan-Salzen der allgemeinen Formel
   R^{a}SiO₃³⁻, R^{a}R^{b}SiO₂²⁻, R^{a}R^{b}R^{c}SiO⁻, R^{a}R^{b}R^{c}SiO₃⁻, R^{a}R^{b}R^{c}SiO₂⁻, R^{a}R^{b}SiO₃²⁻
- den Carbonsäureimiden, Bis(sulfonyl)imiden oder Sulfonylimiden der allgemeinen Formel 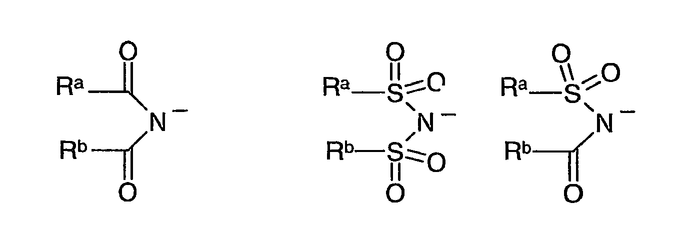
- den Alkoxiden oder Aryloxide der allgemeinen Formel
   R^{a}O⁻
- den komplexen Metallionen wie Fe(CN)₆³⁻, Fe(CN)₆⁴⁻, MnO₄⁻,Fe(CO)₄⁻
- Nitrit; Nitrat, Dicyanamid,
wobei die Reste R^{a}, R^{b}, R^{c} unabhängig voneinander jeweils C₁-C₁₈-Alkyl, C₆-C₁₂-Aryl, C₅-C₁₂-Cycloalkyl, gegebenenfalls durch ein oder mehrere nicht-benachbarte Sauerstoff- und/oder Schwefelatome und/oder ein oder mehrere substituierte oder unsubstituierte Iminogruppen unterbrochenes C₂-C₁₈-Alkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können.

Weitere geeignete Säuren können von den nachfolgenden Anionen [A]ⁿ⁻ abgeleitet werden, wobei das Anion ausgewählt ist aus der Gruppe bestehend aus
- tetrasubstituiertes Borat der allgemeinen Formel [BR^{d}R^{e}R^{f}R^{g}]⁻, wobei R^{d} bis R^{g} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
- (Fluoralkyl)fluorphosphat der allgemeinen Formel [PFₓ(C_{y}F_{2y+1-z}H_{z})₆₋ₓ]⁻, wobei 1 ≤ x ≤ 6, 1 ≤ y ≤ 8 und 0 ≤ z ≤ 2y+1;
- Methid der allgemeinen Formel 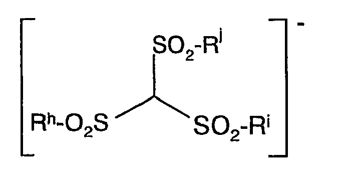 wobei R^{h} bis R^{j} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
- Imidanionen -CO-N⁻-CO-, Sulfonimidanionen -SO₂-N-SO₂- und gemischten Carbosulfoimidanionen -SO₂-N-CO-.

Die Ladung "n-" des Anions [A]ⁿ⁻beträgt "1-", "2-" oder "3-". Als Beispiele zweifach negativ geladener Anionen seien Sulfat, Hydrogenphosphat und Carbonat genannt. Als Beispiel eines dreifach negativ geladenen Anions sei Phosphat genannt.

Die Zugabe der Säure zu der ionischen Flüssigkeit [Q⁺]ₙ[X]ⁿ⁻ kann stöchiometrisch oder titrimetisch bis zu einem pH-Wert erfolgen, der dem Äquivalenzpunkt des entsprechenden Säure-Base-Paares entspricht. Die Säure kann in weiteren Ausführungsformen allerdings auch im Über- oder Unterschuß verwendet werden. So kann die Menge an Säure 0,5 bis 1,5 Äquivalente, vorzugsweise 0,8 bis 1,2 Äquivalente, besonders bevorzugt 0,9 bis 1,1 Äquivalente, insbesondere 1,0 Äquivalent, jeweils bezogen auf die ionische Flüssigkeit [Q⁺]ₙ[X]ⁿ⁻, betragen.

Falls in dem erfindungsgemäßen Verfahren die verwendete, zu modifizierende ionische Flüssigkeit mit einem Alkoholat umgesetzt wird, so bildet sich in der Neutralisation der entsprechende Alkohol. In einer bevorzugten Ausführungsform des erfindungsgemäßen Verfahrens wird daher bei Verwendung von Alkoholaten nach der Neutralisation der in der Neutralisation gebildete Alkohol destillativ entfernt.

Die Neutralisation im zweiten Verfahrensschritt findet bei Temperaturen von vorzugsweise - 10 bis 100°C, besonders bevorzugt 0 bis 90°C, insbesondere 10 bis 60°C, statt. Die Umsetzungsdauer beträgt dabei vorzugsweise 1 bis 16 h, besonders bevorzugt 30 min bis 3 h, insbesondere 10 min bis 2 h.

Der erste und/oder der zweite Verfahrensschritt des erfindungsgemäßen Verfahrens können gegebenenfalls unter Schutzgas, beispielsweise Stickstoff, Edelgase oder Kohlendioxid durchgeführt werden.

Das erfindungsgemäße Verfahren kann kontinuierlich oder diskontinuierlich durchgeführt werden.

Das erfindungsgemäße Verfahren kann in jeder geeigneten, dem Fachmann bekannten Vorrichtung durchgeführt werden. Geeignet sind beispielsweise übliche Vorrichtungen, wie sie in: Kirk-Othmer, Encyclopedia of Chemical Technology, 4. Ed., Vol. 20, John Wiley & Son's, New York 1996, Seiten 1.040 bis 1.055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann dabei auch in mehreren, beispielsweise zwei oder drei, der vorgenannten Vorrichtungen durchgeführt werden.

Im Rahmen der vorliegenden Erfindung versteht man unter dem Begriff der funktionellen Gruppe Gruppen, welche an ein Kohlenstoffatom oder ein Heteroatom gebunden sein können. Als geeignete Beispiele seien -OH (Hydroxy), =O (insbesondere als Carbonylgruppe), -NH₂ (Amino), =NH (Imino), -COOH (Carboxy), -CONH₂ (Carboxamid) und -CN (Cyano) genannt. Fuktionelle Gruppen und Heteroatome können auch direkt benachbart sein, so dass auch Kombinationen aus mehreren benachbarten Atomen, wie etwa -O- (Ether), -S- (Thioether), -COO- (Ester), -CONH- (sekundäres Amid) oder -CONR- (tertiäres Amid), mit umfasst sind.

Das erfindungsgemäße Verfahren eignet sich beispielsweise zur Herstellung von ionischen Flüssigkeiten der allgemeinen Formel [Q⁺] [BR'ₙ(OR")m⁻] mit n = 1, 2, 3 und m =4 - n.

In einer bevorzugten Ausführungsform enthält die ionische Flüssigkeit der allgemeinen Formel [Q⁺] [BRₙ(OR")m⁻] das Anion [BPh₃OR'] mit der für R' zuvor angegebenen Bedeutung.
In einer weiteren bevorzugten Ausführungsform enthält die ionische Flüssigkeit [Q⁺] [BR'ₙ(OR")m⁻] als Anion [BPh₃OR'] und als Kation ein N, N-Dialkylimidazolium-Kation.

Im Rahmen der vorliegenden Erfindung bedeuten
gegebenenfalls durch funktionelle Gruppen, Aryl, Alkyl, Aryloxy, Alkyloxy, Halogen, Heteroatome und/oder Heterocyclen substituiertes C₁ - C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Ethylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, mEthoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Di-oxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxy-ethyl, 2-Hydroxypropyl, 3-Hydroxypropyl, 4-Hydroxybutyl, 6-Hydroxyhexyl, 2-Amino-ethyl, 2-Aminopropyl, 3-Aminopropyl, 4-Aminobutyl, 6-Aminohexyl, 2-Methylamino-ethyl, 2-Methylaminopropyl, 3-Methylaminopropyl, 4-Methylaminobutyl, 6-Methylamino-hexyl, 2-Dimethylaminoethyl, 2-Dimethylaminopropyl, 3-Dimethylaminopropyl, 4-Di-methylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und,
gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befindet sich zwischen zwei Heteroatomen in der Regel mindestens ein Kohlenstoffatom, bevorzugt befinden sich mindestens zwei Kohlenstoffatome zwischen zwei Heteroatomen.

Substituierte und unsubstituierte Iminogruppen können beispielsweise Imino-, Methylimino-, iso-Propylimino, n-Butylimino oder tert-Butylimino sein.

Weiterhin bedeuten
funktionelle Gruppen Carboxy, Carboxamid, Hydroxy, Di-(C₁-C₄-Alkyl)-amino, C₁-C₄-Alkyloxycarbonyl, Cyano oder C₁-C₄-Alkyloxy,
gegebenenfalls durch funktionelle Gruppen, Aryl, Alkyl, Aryloxy, Alkyloxy, Halogen, Heteroatome und/oder Heterocyclen substituiertes C₆-C₁₂-Aryl beispielsweise Phenyl, Tolyl, Xylyl, α-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4-oder 2,6-Dinitrophenyl, 4-Dimethylaminophenyl, 4-Acetylphenyl, Methoxyethylphenyl oder Ethoxymethylphenyl.

Die vorliegende Erfindung stellt ein Verfahren zur universellen Herstellung von ionischen Flüssigkeiten durch Einführung eines nahezu beliebigen Anions bereit. Dabei wird zunächst ein Alkoholat oder Hydroxid einer ionischen Flüssigkeit hergestellt, das anschließend mit einer nahezu beliebigen Säure protoniert und auf diese Weise mit einem Anion versehen werden kann. Dabei ist das erfindungsgemäße Verfahren zur Herstellung einer Vielzahl an unterschiedlichen ionischen Flüssigkeiten geeignet. Im Gegensatz zu den bisher bekannten Verfahren ist das erfindungsgemäße Verfahren kostengünstig durchzuführen.

Die vorliegende Erfindung wird anhand der nachfolgenden Ausführungsbeispiele näher erläutert.

### Ausführungsbeispiele

EMIM = Ethylmethylimidazolium
BMIM = Butylmethylimidazolium

### I. Erfindungsgemäße Versuche (Alkoholatmethode)

### 1. Herstellung von EMIM Acetat

92,6 g (0,825 mol) Kalium tert-Butylat werden in 1000 ml n-Butanol bei 60°C gelöst. Anschließend kühlt man auf RT ab und lößt 120,9 g (0,825 mol) geschmolzenes EMIM Chlorid (Schmp. Ca. 85°C) in die Lösung fließen. Es fällt sofort ein Niederschlag von KCI aus. Das Reaktionsgemisch wird noch 3 h bei RT gerührt und dann filtriert. Der Niederschlag wird mit n-Butanol nachgewaschen. Man erhält 747.2 g Filtrat, das stark alkalisch reagiert (pH 14). Eine Probe des Filtrats wird gegeben 0,5 M HCI titriert. Demnach sind in dem Filtrat 0,666 mol EMIM tert-Butylat enthalten. In das Filtrat wird nun zur Neutralisation des EMIM tert-Butylats die stöichiometrischen Menge = 49,5 g (0,825 mol) Eisessig gegeben. Die Lösemittel werden im Vakuum entfernt. Das zurückbleibende Öl wird zur Entfernung von Spuren möglicherweise überschüssiger Essigsäure mit Ethylacetat extrahiert und im Hochvakuum bei 70°C getrocknet. Es werden 133,7 g (0,785 mol) EMIM Acetat erhalten. Die Ausbeute beträgt 95,2 %. Der Chloridgehalt beträgt 0,52 %, der Wassergehalt 0,04 %.

### 2. Herstellung von EMIM Tosylat

185,2 g (1,65 mol) Kalium tert-Butylat werden in 2000 ml n-Butanol bei 60°C gelöst. Anschließend lässt man 242,0 g (1,65 mol) geschmolzenes EMIM Chlorid (Schmp. Ca. 85°C)in die Lösung fließen. Es fällt sofort ein Niederschlag von KCI aus. Das Reaktionsgemisch wird noch 30 min bei 60°C gerührt und dann filtriert. Der Niederschlag wird mit n-Butanol nachgewaschen. In das Filtrat wird nun zur Neutralisation des E-MIM tert-Butylats p-Toluolsulfonsäure gegeben. Die Lösemittel werden im Vakuum entfernt. Das 1H NMR Spektrum des zurückbleibenden Öls zeigt, dass neben EMIM Tosylat noch 25 % überschüssige p-Toluolsulfonsäure sowie n-Butanol und tert-Butanol enthalten sind.

### 3. Herstellung von EMIM Acetat

119,6 (0,3 mol) EMIM Cl werden als 36.8%ige Lösung in Ethanol bei RT vorgelegt. In diese Lösung lässt man 97,1 g (0,3 mol) einer 21 %igen Natriumethylatlösung fließen. Es fällt sofort ein Niederschlag von NaCl aus. Das Reaktionsgemisch wird noch 30 min bei RT gerührt und dann filtriert. Der Niederschlag wird mit Ethanol nachgewaschen. Man erhält 183,5 g Filtrat, das stark alkalisch reagiert (pH 14). Eine Probe des Filtrats wird gegen 0,5 M HCl titriert. Demnach sind in dem Filtrat 0,271 mol EMIM Ethanolat enthalten. In das Filtrat wird nun zur Neutralisation des EMIM Ethanolats die stöchiometrische Menge = 16,26 g (0,271 mol) Eisessig gegeben. Die Lösemittel werden im Vakuum entfernt. Das zurückbleibende Öl wird zur Entfernung von Spuren möglicherweise überschüssiger Essigsäure mit Ethylacetat extrahiert und im Hochvakuum bei 50°C getrocknet. Es werden 41,3 g (0,24 mol) EMIM Acetat erhalten. Die Ausbeute bezüglich des EMIM Ethanolats beträgt 90 %. Der Chloridgehalt beträgt 5,6 %, der Wassergehalt 0,29 %.

### 4. Herstellung von BMIM Acetat

In 100,0 g (0,309 mol) 21 %ige Natriumethylatlösung werden bei 60°C 53,9 g (0,309 mol) BMIM Chlorid zugegeben. Es fällt sofort ein Niederschlag von NaCl aus. Das Reaktionsgemisch wird noch 2 h bei 60°C gerührt und dann filtriert. Der Niederschlag wird mit Ethanol nachgewaschen. Man erhält 268,3 g Filtrat, das stark alkalisch reagiert (pH14). Eine Probe des Filtrats wird gegen 0,5 M HCl titriert. Demnach sind in dem Filtrat 0,2233 mol BMIM Ethanolat enthalten. In das Filtrat wird nun zur Neutralisation des BMIM Ethanolats die stöchiometrische Menge = 13,2 g (0,22 mol) Eisessig gegeben. Die Lösemittel werden im Vakuum entfernt. Das zurückbleibende Öl wird zur Entfernung von Spuren möglicherweise überschüssiger Essigsäure mit Ethylacetat extrahiert und im Hochvakuum bei 50°C getrocknet. Es werden 41,9 g (0,21 mol) EMIM Acetat erhalten. Die Ausbeute bezüglich des BMIM Ethanolats beträgt 95 %. Der Chloridgehalt beträgt 0,59%.

### 5. Herstellung einer ionischen Flüssigkeiten durch Umsetzung einer basischen ionischen Flüssigkeit mit einer Lewissäure

9 g (0,08 mol) Kalium-tert-butanolat werden in 100 ml n-Butanol bei 60°C gelöst. Nach Abkühlen auf RT werden 11,7 g (0,08 mol) EMIM Cl als Schmelze zugegeben. Man rührt noch 3 Stunden bei RT und filtriert dann das ausgefallene KCl ab und spült mit n-Butanol nach. Es werden 97,9 g Mutterlauge erhalten, die laut Titration gegen 0,5 M HCl 0,076 mol Butanolat enthalten, was einer Ausbeute in diesem Schritt von 95% entspricht. 18,5 g (0,076 mol) Triphenylbor werden in 100 ml n-Butanol gelöst. Anschließend tropft man die 97,9 g der butanolischen Lösung des EMIM Butanolats in die Lösung des Triphenylbor. Eine exotherme Reaktion wird beobachtet. Nach vollständiger Zugabe wird das Lösemittel im Vakuum entfernt. Es bleiben 30,8 g ionische Flüssigkeit zurück, was einer Ausbeute von 95% entspricht. Laut ¹H NMR handelt es sich um die ionische Flüssigkeit EMIM Triphenyl-n-butoxyborat. Das zunächst eingesetzte tert-Butanolat hat sich in überschüssigem n-Butanol, das als Lösemittel eingesetzt wurde, in n-Butanolat umgewandelt. Das ¹H NMR Spektrum (CDCl₃) zeigt die entsprechenden Signale bei 7,5 ppm (m, 6H, *o-*Ph-*H*), 7,25 ppm (1H, N-C*H*-N), 7,0 (m, 6H, *m*-Ph-*H*), 6,9 (m, 3H, *p*-Ph-*H*), 6,35 ppm (s, 1H, N-C*H*-CH-N), 6,25 ppm (s, 1H, N-CH-C*H*-N), 3,62 (t, 2H, OC*H*₂CH₂CH₂CH₃), 3,4 (q, 2H, CH₃C*H*₂N), 2,95 ppm (s, 3H, N-C*H*₃), 1,5 (m, 2H, OCH₂C*H*₂CH₂CH₃), 1,4 (m, 2H, OCH₂CH₂C*H*₂CH₃), 1,05 (t, 3H, C*H*₃CH₂N), 0,9 (t, 3H, OCH₂CH₂CH₂C*H*₃). Im Bor NMR ist das Signal eines Borates bei ca. -0,5 ppm zu sehen. Es liegt also kein freies Triphenylbor mehr vor, das bei 67 ppm liegen würde. Die ionische Flüssigkeit hat einen Schmelzpunkt von 99°C, nach Umkristallisieren aus Ethylacetat von 105°C.

### II. Erfindungsgemäβe Versuche (Bariummethode)

### 1. Herstellung von EMIM Acetat

220,8 g (0,72 mol; enthält noch überschüssige H₂SO₄) EMIM HSO₄, werden in 600 ml Wasser gelöst. 523,8 g (1,66 mol) BaOH₂*8H₂O werden innerhalb von 30 min bei RT in Portionen zugegeben. Die Temperatur wird auf 60°C erhöht und 2 Stunden nachgerührt. Man lässt über Nacht abkühlen und filtriert das ausgefallene BaSO₄ mit Celite als Filterhilfsmittel über eine Nutsche ab. Es werden 995,4 g Filtrat erhalten, das lt. Titration gegen 0,5 M HCl 0,65 mol EMIM OH enthält. 43,5 g (0,72 mol) Eisessig werden zugegeben. Wasser wird am Rotationsverdampfer entfernt, das zurückbleibende Öl mit Ethylacetat extrahiert. Um Wasserreste zu entfernen, wird das Öl mit n-Butanol versetzt und dieses anschließend im Vakuum abdestilliert. Man erhält 108,3 g (0,636 mol) E-MIM Acetat. Die Ausbeute in Bezug auf EMIM OH beträgt 98 %. Die Ausbeute in Bezug auf EMIM HSO₄ beträgt 88 %. Bei dem Öl handelt es sich lt. 1H NMR um die ionische Flüssigkeit EMIM Acetat. Der Chloridgehalt beträgt 180 ppm, der Schwefelgehalt 160 ppm, der Bariumgehalt 650 ppm, der Wassergehalt 0,68 %.

### 2. Herstellung von EMIM Acetat

453,5 g (1,441 mol) BaOH₂*8H₂O werden in 600 g Wasser suspendiert. Man heizt auf 80°C auf. Das Bariumsalz schmilzt bei ca. 80°C und liegt nun als Emulsion mit Wasser vor. In die Emulsion tropft man 220,8 g (0,72 mol; enthält überschüssige H₂SO₄) EMIM HSO₄, wobei die Temperatur auf 100°C ansteigt. Trotz des ausgefallen BaSO₄ bleibt die Suspension gut rührbar. Nach 2 Stunden Nachrühren bei 80°C ist das Filtrat sulfatfrei (negativer Sulfattest). Nach Abkühlen filtrier man das ausgefallene BaSO₄ mit Celite als Filterhilfsmittel über eine Nutsche ab. Das Filtrat wird mit 43,5 g Eisessig (0,72 mol) versetzt. Wasser wird am Rotationsverdampfer entfernt. Das zurückbleibende Öl wird mit Ethylacetat extrahiert. Nach Trocknen im Vakuum erhält man 113,3 g (0,67 mol) EMIM Acetat. Die Ausbeute beträgt 92 %. Der Chloridgehalt beträgt 60 ppm, der Wassergehalt 0,67 %.

### 3. Herstellung von EMIM Acetat

403,8 g (1,28 mol) BaOH₂*8H₂O werden bei 75°C mit 76,9 g (1,28 mol) Eisessig in 350 g Wasser vorgelegt, wobei eine Lösung erhalten wird. In die Lösung tropft man 266,5 g (1,28 mol) EMIM HSO₄, wobei die Temperatur auf 86°C ansteigt. Trotz des ausgefallenen BaSO₄ bleibt die Suspension gut rührbar. Unter Abkühlen rührt man 90 min nach und filtiriert das ausgefallene BaSO₄ mit Celite als Filterhilfsmittel über eine Nutsche ab. Das Filtrat wird am Rotationsverdampfer eingeengt. Zur Trocknung setzt man noch n-Butanol zu und destilliert erneut ab. Das 1H NMR Spektrum zeigt reines EMIM Acetat.

### 4. Herstellung von EMIM Dihydrogenphosphat

237,57 g (1,0 mol; enthält noch überschüssige H₂SO₄) werden in 600 ml Wasser gelöst. 473,3 g (1,5 mol) BaOH₂*8H₂O werden innerhalb von 30 min bei RT in Portionen zugegeben. Die Temperatur wird auf 60°C erhöht und 2 Stunden nachgerührt. Man lässt über Nacht abkühlen und filtriert das ausgefallene BaSO₄ mit Celite als Filterhilfsmittel über eine Nutsche ab und wäscht mit Wasser nach. Es werden 1320,5 g Filtrat erhalten, das lt. Titration gegen 0,5 M HCl 0,93 mol EMIM OH enthält. 115,3 g (1,0 mol) 85 %ige Phosphorsäure werden zugegeben. Wasser wird am Rotationsverdampfer entfernt. Der Rückstand (202,8 g = 0,975 mol) ist ein weißer Feststoff mit einem Schmelzpunkt von 140°C. Die Ausbeute in Bezug auf EMIM HSO₄ beträgt 98 %. Bei dem Feststoff handelt es sich lt. ¹H NMR um EMIM Dihydrogenphosphat. Der Chloridgehalt beträgt 550 ppm, der Wassergehalt 1,4 %.

### 5. Herstellung von EMIM Saccharinat

315,5 g (1,0 mol) BaOH₂*8H₂O und 186,9 g (1 mol) Saccharin werden in 1000 ml Wasser bei 75°C suspendiert. In die Mischung tropft man innerhalb von 30 min 208,2 g (1,0 mol) EMIM HSO₄, wobei die Suspension dünnflüssiger wird. Nach 30 min Nachrühren war das Filtrat bereits sulfatfrei (negativer Sulfattest). Man filtriert das ausgefallene BaSO₄ mit Celite als Filterhilfsmittel über eine Nutsche ab und wäscht mit Wasser nach. Wasser wird am Rotationsverdampfer entfernt. Der Rückstand (nach Trocknen im Vakuum 285 g = 0,971 mol) ist ein Feststoff, bei dem es sich lt ¹H NMR um EMIM Saccharinat handelt. Der Wassergehalt beträgt 0,3 %. Die Ausbeute beträgt 97 %. Der Schmelzpunkt von EMIM Saccharinat beträgt ca. 150°C.

### 6. Herstellung von EMIM Dihydrogenborat

631 g (2,0 mol) BaOH₂*8H₂O und 123,6 g (2 mol) Borsäure werden in 500 ml Wasser bei 60°C suspendiert. In die Mischung tropft man innerhalb von 60 min 416,4 g (1,0 mol) EMIM HSO₄. Zur besseren Rührbarkeit werden weitere 500 g Wasser zugegeben. Man filtriert das ausgefallene BaSO₄ mit Celite als Filterhilfsmittel über eine Nutsche ab und wäscht mit Wasser nach. Wasser wird mit zugesetztem n-Butanol am Rotationsverdampfer entfernt. Der Rückstand (nach Trocknen im Vakuum 247,2 g = 1,44 mol) ist ein Feststoff, der lt. ¹H NMR das EMIM Kation enthält. Die Ausbeute beträgt 72 %. Der Schmelzpunkt von EMIM Dihydrogenborat beträgt ca.40°C.

### 7. Herstellung von EMIM Cyanurat

315,5 g (1,0 mol) BaOH₂*8H₂O und 129,1 g (1 mol) Cyanursäure werden in 500 ml Wasser bei 60°C suspendiert. In die Mischung tropft man innerhalb von 35 min 208,2 g (1,0 mol) EMIM HSO₄. Nach 8 Stunden Nachrühren wird abgekühlt. Man filtriert das ausgefallene BaSO₄ mit Celite als Filterhilfsmittel über eine Nutsche ab. Wasser wird mit zugesetztem n-Butanol am Rotationsverdampfer entfernt. Der Rückstand (nach Trocknen im Vakuum 197,5 g = 0,825 mol) ist ein Feststoff, der lt 1H NMR das EMIM Kation enthält. Der Chloridgehalt beträgt 0,23 %. Die Ausbeute beträgt 83 %. Der Schmelzpunkt von EMIM Cyanurat beträgt ca. 161°C.

## Patentansprüche

1. Verfahren zur Modifizierung ionischer Flüssigkeiten, die als Kation ein Phosphonium- und/oder Ammoniumkation, das aus der nachfolgend dargestellten Gruppe ausgewählt ist, nämlich aus
- quartemären Ammonium-Kationen der allgemeinen Formel
[NR¹R²R³R]⁺,
- Phosphonium-Kationen der allgemeinen Formel
[PR¹R²R³R]⁺,
- Imidazolium-Kationen der allgemeinen Formel 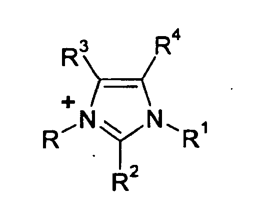 sowie allen dieser Formel analogen isomeren Imidazoliniumkationen und Imidazolidiniumkationen,
- H-Pyrazolium-Kationen der allgemeinen Formel 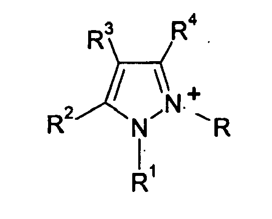 sowie 3H-Pyrazolium-Kationen, 4H-Pyrazolium-Kationen, 1-Pyrazolinium-Kationen, 2-Pyrazolinium-Kationen und 3-Pyrazolinium-Kationen,
- Pyridinium-Kationen der allgemeinen Formel 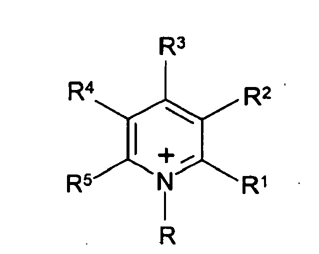 sowie Pyridazinium-, Pyrimidinium- und Pyrazinium-Kationen,
- Pyrrolidinium-Kationen der allgemeinen Formel 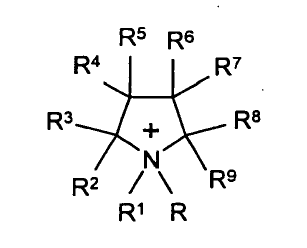
- fünf- bis mindestens sechsgliedrigen heterocyclischen Kationen, die mindestens ein Phosphor- oder Stickstoffatom sowie gegebenenfalls ein Sauerstoff- oder Schwefelatom aufweisen,
- Triazol-Kationen der allgemeinen Formel 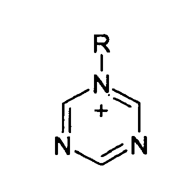 wobei der Triazol-Kern substituiert sein kann mit wenigstens einer Gruppe, die ausgewählt ist aus der Gruppe bestehend aus C₁-C₆-Alkyl-, C₁-C₆-Alkoxy-, Amino-C₁-C₆-alkyl-, C₅-C₁₂-Aryl- oder C₅-C₁₂-Aryl-C₁-C₆-alkylgruppen,
- dem 1,8-Diazabicyclo[5.4.0]undec-7-enium-Kation sowie dem 1,8-Diazabicyclo₋[4.3.0]non-5-enium-Kation 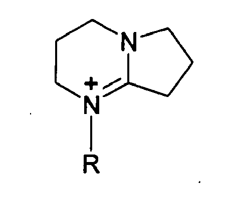
- Chinolinium-Kationen der allgemeinen Formel 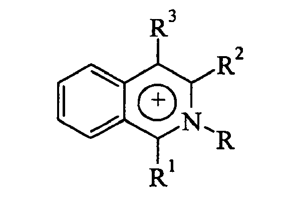
- Thiazolium-Kationen der allgemeinen Formel 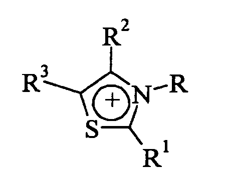
- Triazinium-Kationen der allgemeinen Formel 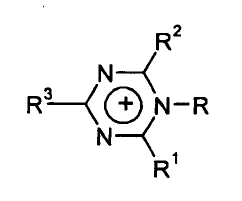 sowie Oligo- und Polymere, die diese Kationen enthalten, wobei die Reste R, R¹, R², R³, R⁴, R⁵, R⁶, R⁷, R⁸ und R⁹ unabhängig voneinander jeweils Wasserstoff, C₁-C₁₈-Alkyl, C₆-C₁₂-Aryl, C₅-C₁₂-Cycloalkyl, C₂-C₁₈-Alkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigen oder aromatischen und gegebenenfalls durch ein oder mehrere Sauerstoff- und/oder Schwefelatome und/oder ein oder mehrere subsituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen, Heteroatome und/oder Heterocyclen substituiert sein können und ein Anion, das ausgewählt ist aus der Gruppe, bestehend aus Halogeniden, Arylsulfonaten, Alkylsulfonaten, Sulfat, Hydrogensulfat, Alkylsulfaten, Hydrogencarbonat, Carbonat, Triflaten und Carboxylaten, enthält, **dadurch gekennzeichnet, dass** diese ionischen Flüssigkeiten in einem ersten Verfahrensschritt mit einem Alkoholat oder Bariumhydroxid bei 10 bis 90 °C umgesetzt werden, wobei stark basische ionische Flüssigkeiten resultieren, und die stark basischen ionischen Flüssigkeiten in einem zweiten Verfahrensschritt mit einer Säure neutralisiert werden.

2. Verfahren nach Anspruch 1, **dadurch gekennzeichnet, dass** bei Einsatz von Alkoholaten nach der in dem zweiten Verfahrensschritt erfolgenden Neutralisation in der Neutralisation gebildeter Alkohol destillativ entfernt wird.

3. Verfahren nach Anspruch 1 oder 2, **dadurch gekennzeichnet, dass** nach dem ersten Verfahrensschritt der ausgefallene Feststoff abgetrennt wird.

4. Verfahren nach einem der Ansprüche 1 bis 3, **dadurch gekennzeichnet, dass** die ionische Flüssigkeit ein heterocyclisches Kation enthält.

5. Verfahren nach Anspruch 4, **dadurch gekennzeichnet, dass** die ionische Flüssigkeit ein Imidazolium-Kation enthält.

6. Verfahren nach einem der Ansprüche 1 bis 5, **dadurch gekennzeichnet, dass** in dem zweiten Verfahrensschritt eine Neutralisation der stark basischen ionischen Flüssigkeit mit einer Säure bis zu einem pH-Wert erfolgt, der dem Äquivalenzpunkt des entsprechenden Säure-Base-Paares entspricht.

7. Verfahren nach einem der Ansprüche 1 bis 6, **dadurch gekennzeichnet, dass** die Umsetzung in dem zweiten Verfahrensschritt bei einer Temperatur von -10 bis 100°C stattfindet.

## Claims

1. A process for modifying ionic liquids containing a phosphonium and/or ammonium cation which is selected from the following group, namely from among
- quaternary ammonium cations from the general formula
[NR¹R²R³R]⁺,
- phosphonium cations of the general formula
[PR¹R²R³R]⁺,
- imidazolium cations of the general formula 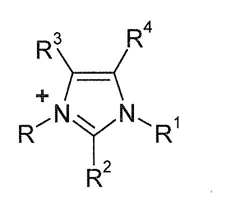 and also all isomeric imidazolinium cations and imidazolidinium cations analogous to this formula,
- H-pyrazolium cations of the general formula 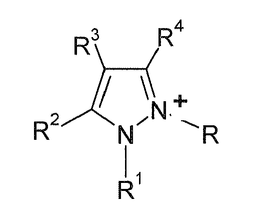 and also 3H-pyrazolium cations, 4H-pyrazolium cations, 1-pyrazolinium cations, 2-pyrazolinium cations and 3-pyrazolinium cations,
- pyridinium cations of the general formula 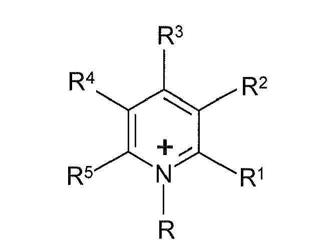 and also pyridazinium, pyrimidinium and pyrazinium cations,
- pyrrolidinium cations of the general formula 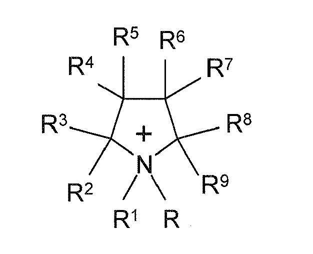
- five- to at least six-membered heterocyclic cations which contain at least one phosphorus or nitrogen atom and optionally an oxygen or sulfur atom,
- triazole cations of the general formula 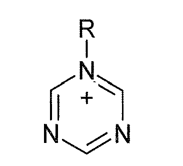 where the triazole ring can be substituted by at least one group selected from the group consisting of C₁-C₆-alkyl, C₁-C₆-alkoxy, amino-C₁-C₆-alkyl, C₅-C₁₂-aryl or C₅-C₁₂-aryl-C₁-C₆-alkyl groups,
- the 1,8-diazabicyclo[5.4.0]undec-7-enium cation and also the 1,8-diazabicyclo-[4.3.0]non-5-enium cation 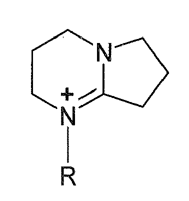
- quinolinium cations of the general formula 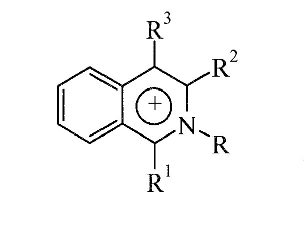
- thiazolium cations of the general formula 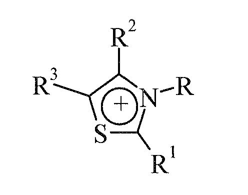
- triazinium cations of the general formula 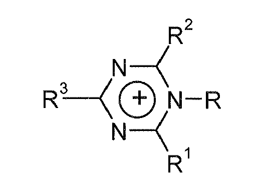 and also oligomers and polymers in which these cations are present, where the radicals R, R¹, R², R³, R⁴, R⁵, R⁶, R⁷, R⁸ and R⁹ are each, independently of one another, hydrogen, C₁-C₁₈-alkyl, C₆-C₁₂-aryl, C₅-C₁₂-cycloalkyl, C₂-C₁₈-alkyl or a five- or six-membered, oxygen-, nitrogen- and/or sulfur-containing heterocycle or two of them together form an unsaturated, saturated or aromatic ring which may be interrupted by one or more oxygen and/or sulfur atoms and/or one or more substituted or unsubstituted imino groups, where each of the radicals mentioned may be substituted by functional groups, aryl, alkyl, aryloxy, alkyloxy, halogen, heteroatoms and/or heterocycles, as cation and an anion selected from the group consisting of halides, arylsulfonates, alkylsulfonates, sulfate, hydrogensulfate, alkylsulfates, hydrogencarbonate, carbonate, triflates and carboxylates, wherein these ionic liquids are reacted with an alkoxide or barium hydroxide at from 10 to 90°C in a first process step, resulting in strongly basic ionic liquids, and the strongly basic ionic liquids are neutralized with an acid in a second process step.

2. The process according to claim 1, wherein alcohol formed in the neutralization when alkoxides are used is removed by distillation after the neutralization carried out in the second process step.

3. The process according to claim 1 or 2, wherein the precipitated solid is separated off after the first process step.

4. The process according to any of claims 1 to 3, wherein the ionic liquid contains a heterocyclic cation.

5. The process according to claim 4, wherein the ionic liquid contains an imidazolium cation.

6. The process according to any of claims 1 to 5, wherein, in the second process step, the strongly basic ionic liquid is neutralized with an acid to a pH corresponding to the equivalence point of the corresponding acid-based pair.

7. The process according to any of claims 1 to 6, wherein the reaction in the second process step takes place at a temperature of from -10 to 100°C.

## Revendications

1. Procédé pour la modification de liquides ioniques, qui contiennent comme cation un cation phosphonium et/ou ammonium, qui est choisi dans le groupe présenté ci-après, à savoir parmi
- des cations ammonium quaternaire de formule générale
[NR¹R²R³R]⁺,
- des cations phosphonium de formule générale
[PR¹R²R³R]⁺,
- des cations imidazolium de formule générale 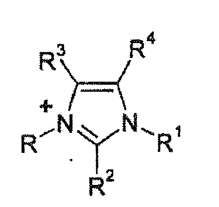 ainsi tous les cations imidazolinium et cations imidazolidinium isomères analogues à cette formule,
- des cations H-pyrazolium de formule générale 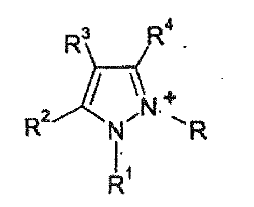 ainsi que des cations 3H-pyrazolium, des cations 4H-pyrazolium, des cations 1-pyrazolinium, des cations 2-pyrazolinium, et des cations 3-pyrazolinium,
- des cations pyridinium de formule générale 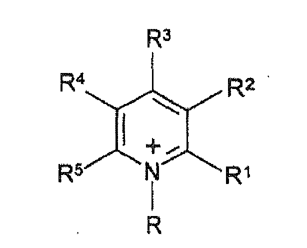 ainsi que des cations pyridazinium, pyrimidinium et pyrazinium,
- des cations pyrrolidinium de formule générale 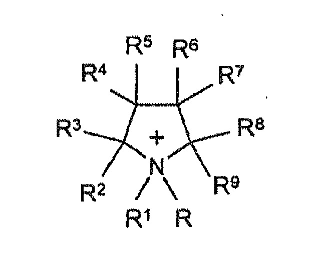
- des cations hétérocycliques à cinq à au moins six chaînons, qui comportent au moins un atome de phosphore ou d'azote, ainsi qu'éventuellement un atome d'oxygène ou de soufre,
- des cations triazole de formule générale 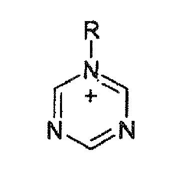 le noyau triazole pouvant être substitué par au moins un groupe qui est choisi dans l'ensemble constitué par des groupes alkyle en C₁-C₆, alcoxy en C₁-C₆, amino-alkyle(C₁-C₆), aryle en C₅-C₁₂ ou aryl(C₅-C₁₂)-alkyle(C₁-C₆),
- le cation 1,8-diazabicyclo[5.4.0]undéc-7-énium ainsi que le cation 1,8-diazabicyclo[4.3.0]non-5-énium 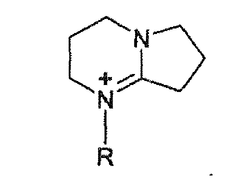
- des cations quinolinium de formule générale 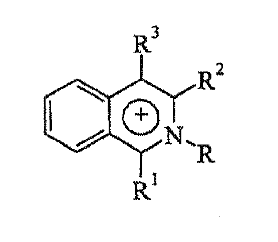
- des cations thiazolium de formule générale 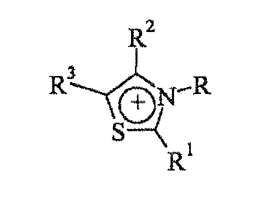
- des cations triazinium de formule générale 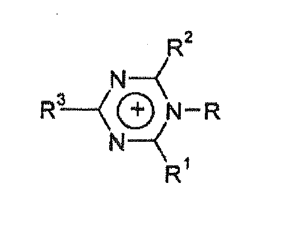 ainsi que des oligo- et polymères qui contiennent ces cations, les radicaux R, R¹, R², R³, R⁴, R⁵, R⁶, R⁷, R⁸ et R⁹ représentant chacun, indépendamment les uns des autres, un atome d'hydrogène, un groupe alkyle en C₁-C₁₈, aryle en C₆-C₁₂, cycloalkyle en C₅-C₁₂, alkyle en C₂-C₁₈ ou un hétérocycle à cinq ou six chaînons, comportant des atomes d'oxygène, d'azote et/ou de soufre, ou deux de ces radicaux formant ensemble un cycle insaturé, saturé ou aromatique et éventuellement interrompu par un ou plusieurs atomes d'oxygène et/ou de soufre et/ou un ou plusieurs groupes imino substitués ou non substitués, les radicaux nommés pouvant être substitués par des groupes fonctionnels, des groupes aryle, alkyle, aryloxy, des atomes d'halogène, des hétéroatomes ou des hétérocycles, et un anion qui est choisi dans le groupe constitué par des halogénures, arylsulfonates, alkylsulfonates, l'ion sulfate, hydrogénosulfate, des alkylsulfates, l'ion hydrogénocarbonate, carbonate, des triflates et carboxylates, **caractérisé en ce que** dans une première étape on fait réagir ces liquides ioniques avec un alcoolate ou de l'hydroxyde de baryum, à une température de 10 à 90 °C, de sorte qu'il en résulte des liquides ioniques fortement basiques, et dans une deuxième étape du procédé on neutralise par un acide les liquides ioniques fortement basiques.

2. Procédé selon la revendication 1, **caractérisé en ce que** lorsqu'on utilise des alcoolates selon la neutralisation à effectuer dans la deuxième étape du procédé on élimine par distillation l'alcool formé dans la neutralisation.

3. Procédé selon la revendication 1 ou 2, **caractérisé en ce qu'**après la première étape du procédé on sépare le solide précipité.

4. Procédé selon l'une quelconque des revendications 1 à 3, **caractérisé en ce que** le liquide ionique contient un cation hétérocyclique.

5. Procédé selon la revendications 4, **caractérisé en ce que** le liquide ionique contient un cation imidazolium.

6. Procédé selon l'une quelconque des revendications 1 à 5, **caractérisé en ce que** dans la deuxième étape du procédé on effectue une neutralisation par un acide du liquide fortement ionique, jusqu'à un pH qui correspond au point d'équivalence de la paire acide-base correspondante.

7. Procédé selon l'une quelconque des revendications 1 à 6, **caractérisé en ce que** la deuxième étape du procédé a lieu à une température de -10 à 100°C.
